# EUROPEAN PATENT SPECIFICATION

(11) **EP 2 566 548 B1**
(45) Date of publication and mention of the grant of the patent: **13.04.2016**
(21) Application number: 11720617.7
(22) Date of filing: 13.04.2011
(51) Int. Cl.: A61M 15/00

(54) **INHALER COMPRISING BLISTER PACKAGE**
INHALATOR MIT BLISTERVERPACKUNG
INHALATEUR COMPRENANT UN EMBALLAGE-COQUE

(30) Priority: 13.04.2010 TR 201002877
(43) Date of publication of application: 13.03.2013
(73) Proprietor: Sima Patent ve Lisanslama Hizmetleri Ltd.Sti., Esenler/Istanbul (TR)
(72) Inventor: BILGIC, Mahmut, D Blok 34220 Esenler-ISTANBUL (TR)
(86) International application number: PCT/TR2011/000091
(87) International publication number: WO 2011/129791

(56) References cited:
- EP-A1- 2 082 759
- WO-A1-02/36189
- WO-A1-2009/003989
- US-A1- 2009 078 252

## Description

### Field of the Invention

The present invention relates to an inhaler used for delivering a drug in dry powder form to a patient by the inhalation route. In addition, the present invention relates to an inhaler providing safe and effective delivery of a dry powder drug stored in blister packages and the method for using said device.

### Description of the Prior Art

It is well known to use inhalers for delivering medicaments utilized in the treatment and prophylaxis of respiratory diseases by the inhalation route. Inhalation treatment is the most commonly preferred treatment method in these diseases as the inhalers provide ease of use; the medicaments have a more rapid onset time resulting from local administration and they have fewer side effects. Inhalers have been designed in order to provide effective and sufficient delivery of the medicaments used in the treatment of respiratory diseases, particularly in asthma and chronic obstructive pulmonary disease. These inhalers vary according to their operating mechanisms and the physical form of the medicament to be delivered.

In the inhalers used to deliver drugs in dry powder form, the drug is carried in reservoirs, capsules or blisters packages. It is important to deliver each dose to the patient with exact accuracy and preciseness since the required medicament dose in the inhalation is very low.

Inhalation devices comprising blister packages, which are one of the inhaler types utilized for the inhalation of the medicaments in dry powder form mentioned above, are commonly used. The blister package is comprised of a plurality of blister pockets each of which contains medicament in dry powder form. In response to each actuation of the inhaler to realize the inhalation, the blister package is indexed from one part of the device to the other; one of the blister pockets is opened and one dose of the dry powder medicament becomes ready for inhalation subsequent to the blister package being perforated by piercing components in the device or being peeled. The dry powder medicament is provided to be entrained with the air entering the blister pocket which is opened upon the inhalation of the patient and reach the lungs passing through the airways of the patient. Thanks to the blister packages they comprise, this type of inhaler can be used repeatedly at certain intervals without requiring any additional operations to place the blister pocket containing one dose of the medicament in dry powder form before inhalation and the moisture effects on the dry powder medicament in the blisters are prevented in this way. In addition, blister packages enable the dry powder medicament to last for long periods of time by protecting it from gasses such as oxygen, which affect the transmission characteristics of the device, as well as environmental factors such as UV rays.

It is highly significant to deliver a sufficient amount of the dry powder medicament contained in the blister pocket which is peeled to realize an effective inhalation in the inhalers comprising blister packages to the lungs of the patient in each inhalation. The mechanism and the specifications of the inhaler are of the most important factors affecting the efficiency of the inhalation, in addition to the dry powder medicament's physical and chemical characteristics such as suitable aerodynamic particle size, suitable particle shape, uniformity of the particle size distribution, low aerodynamic dispersion forces, low density, high physical and chemical stability. The strong effect of the medicaments, which are used in inhalation treatment, at low doses highlights the significance of controlled dosing in each inhalation during the inhalation treatment.

Underdosing of the dry powder medicament administered to the patient during inhalation operation results in the absence of desired effects while overdosing results in undesirable side effects. Thus, it is required that the necessary amount of the dry powder medicament is delivered to the patient during inhalation for the desired effect to be observed in the treatment.

A wide range of inhalers have been designed to enable inhalation of the medicament in dry powder form from blisters.

WO 2009/003989 discloses an inhaler suitable for delivery of the medicament in dry powder form from a blister package composed of a plurality of blister pockets. Said device comprises a mouthpiece, a gear mechanism, a rotatable mouthpiece cover, a drive gear, a housing and a pawl to prevent movement of the mouthpiece cover. The application aims to provide an inhalation device wherein a fault indicator that operates with the help of a DC motor has been placed.

EP2082759 discloses an inhaler device comprising wherein a mouthpiece cover can be opened up to a certain degree without actuating the device with the help of a freewheel.

US 2009/078252 discloses a fault sensing system for dry powder inhalers. The document mainly aims to detect breakage or some other sort of failure (such as stretching or delamination or tearing) of the blister strip to prevent the user from using the device although it no longer delivers the dry powder.

WO02/36189 discloses a medicament dispenser comprising; a body, a holder shaped to fit within said body and moveable relative to the body, a cassette containing medicament carrierwherein movement of the holder relative to the body results in movement of the cassette between a first position and a second position such that the cassette is reversibly removable from the holder when the cassette is in the second position.

The inhalation device marketed under the trade mark Diskus® by GlaxoSmithKlein is one of the most well-known inhalers on the market. In Diskus® inhaler, each blister pocket of the blister package is prepared for inhalation with the slide mechanism. Before each inhalation, the mouthpiece cover is slid and the mouthpiece and the slide are exposed. In this device, the lever attached to the slide triggers the gear mechanism while the slide is being rotated from one end to the other; the blister package is indexed in the device; the blister pocket is opened and the medicament in dry powder form becomes ready for inhalation.

There seems a need for an inhaler which guarantees controlled dosing of the dry powder medicament in the peeled blister pocket in the inhaler comprising blister packages when current inhalers are taken into consideration.

According to this, the inventor has designed an inhaler which has a mechanism operating with minimum margin of error in order to guarantee the inhalation of the required dose of the medicament in dry powder form in each inhalation and provide the accurate positioning of the blister package.

### Summary of the Invention

An inhaler suitable for delivery of the medicament in dry powder form according to the present invention comprises:
a blister package composed of a plurality of blister pockets which are spaced at equal intervals, each of which comprises medicament in dry powder form;
a mouthpiece enabling the patient to inhale the medicament in dry powder form from the opened blister;
a gear mechanism enabling the blister package to index and the medicament in dry powder form to become ready for inhalation;
a rotatable mouthpiece cover covering the mouthpiece and triggering the gear mechanism;
a drive gear which is directly joined with the mouthpiece cover; and
a housing situated between the upper housing member and the lower housing member in which the blister package and the gear mechanism are enclosed;
wherein an inside lock connection is arranged between the mouthpiece cover and the drive gear by the two ends of the drive gear that enables the mouthpiece cover to connect with the gear mechanism engaging with the recessed parts on the inside face of the mouthpiece cover through a male-female connection.

The two ends of the drive gear which engage with the recessed parts on the inside face of the mouthpiece cover through a male-female connection enable an inside lock connection to be provided between the mouthpiece cover and the drive gear. So that, the inside lock connection formed between the mouthpiece cover and the drive gear provides the blister package to be accurately positioned as a result of the exact transmission of the movement of the mouthpiece cover to the other components of the gear mechanism via the drive gear in response to each actuation of the device.

The inhaler pertaining to the present invention is an easy-grip, manual device which is suitable to be used for the delivery of the medicament in dry powder form.

The housing of the device pertaining to the present invention has been designed such that each component of the blister package and the gear mechanism which have a significant role in enabling the device to work properly is situated accurately and works harmoniously. To this end, the housing is divided into several compartments. The used portion and the unused portion of the blister package are accommodated in separated compartments in order to prevent the residual dry powder medicament in the opened blister pocket spilling on the other components of the housing. Furthermore, the housing also comprises a beak which enables the blister package to be peeled and a manifold through which the dry powder medicament in the open blister pocket passes before reaching the mouthpiece during the inhalation. The housing can be in any appropriate shape through it is preferably elliptic or circular.

The upper and the lower housing members interlock with each other and enclose the housing in order to keep the housing and the gear mechanism fixed together. The mouthpiece cover hiding the mouthpiece is rotated by sliding on the upper and lower housing members. The upper and the lower housing members can be in any appropriate shape which provides ease of use.

The mouthpiece cover hiding the mouthpiece of the device pertaining to the present invention has also been designed such that it actuates the device. When the upper and lower housing members are joined together, engagement tabs on the inside surface of the lower housing member engage with engagement recesses on the inside surface of the upper housing member and the upper and lower housing members are fixed tightly.In addition, the protrusions on the upper and the lower housing members are joined end to end and they form a restricted path along which the mouthpiece cover rotates.Before each inhalation, both the mouthpiece is uncovered and one dose of the medicament in dry powder becomes ready for inhalation as one of the blister pockets is opened as a result of the mouthpiece cover's preferably being manually rotated along the path restricted by the joining of the protrusions on the upper and the lower housing members.The rotational path on which the cover moves is restricted on both ends by the protrusions of the upper and the lower housing members. The constant-distance path that the protrusions of the upper and the lower housing members define allows the mouthpiece cover to be rotated by a fixed angle in the range of30° to 160°, preferably, 50°to 120°, most preferably 55°,60°,65°,70°,75°,80° to 92.5°,95°,97.5°,100°,102.5°,105°,107.5°,110°,112.5°,115° according to the shape of the device in response to the each actuation of the device.

The mouthpiece cover that triggers the gear mechanism of the device can be found in two positions. When the mouthpiece cover is in the first position, the mouthpiece cover resides on the protruding part on one end of the rotational path. When the first position is on, the mouthpiece cover is completely covered and the device is in standby mode. When the mouthpiece is in the second position, it resides on the protruding part on the other end of the rotational path and one dose of the dry powder medicament becomes ready for inhalation upon the actuation of the device.

According to the present invention, there preferably exists a finger tab on the device cover. The word "finger tab" refers to the component that enables the patient to slide the cover comfortably.

Each gear of the gear mechanism in the device according to the present invention directly or indirectly engages with each other. The drive gear, which is one of the components of the gear mechanism, provides the mouthpiece cover to trigger the gear mechanism in such a way that the two ends of the drive gear which engage with the recessed parts on the inside face of the mouthpiece cover through a male-female connection enable an inside lock connection to be provided between the mouthpiece cover and the drive gear. One of the recesses on the inside surface of the mouthpiece cover engages with the one end of drive gear while the other recess on the inside surface of the mouthpiece cover engages with the other end of the drive gear and the connection, that is provided between the each end of the drive gear and the corresponding recess on the inside surface of the mouthpiece cover, is a male-female connection.

As regards the other components of the gear mechanism, in each actuation of the device, the constant-angle rotational movement of the cover is transmitted by the indexing ratchet wheel which interlocks with the indexing wheel via the drive gear. The indexing wheel synchronizes with the indexing ratchet wheel when the mouthpiece cover is switched from the first position to the second position. The gear of the indexing wheel is engaged with the winding wheel gear and the pinion gear and the rotation of the indexing wheel gear causes them to move as well. The mechanism wheel engages with the winding wheel gear via its arms. The counter gear that engages with the small gear under the base gear rotates by means of the base gear that engages with the pinion gear and counter gear with movement of the mouthpiece cover. Therefore, with the rotation of the indexing wheel, both the lid sheet of the blister package is provided to be coiled on the wings of the winding wheel as the winding wheel is rotated by the mechanism wheel that engages with the winding wheel gear, and the counter gear that engages with the small gear under the base gear rotates by means of the base gear that engages with the pinion gear. After the counter gear is rotated, the new number of unused blister pockets can be seen through the display aperture. According to the present invention, the mouthpiece cover triggers the gear mechanism via the drive gear. Therefore, the rotation of the mouthpiece cover has to be transmitted to the drive gear precisely in order to prevent uncontrolled dosing resulting from the mispositioning of the blister package when the device is triggered. In an inhalation device comprising a blister package which has a gear mechanism triggered by the mouthpiece cover, it is required that the rotation of the mouthpiece cover is transmitted to the gear mechanism with minimum margin of error for the blister package to be accurately positioned.

In each actuation of the inhalation device, if the blister package is not indexed or positioned properly then the blister pocket is not opened completely and as a result less than the required amount of the dry powder medicament is inhaled; or if more than one blister pocket is opened more than the required amount of the dry powder medicament is inhaled, which may result in dangerous complications for the patient. Therefore, it is highly significant to provide the blister to be positioned at accurate position and to be completely opened in each actuation of the inhalation device.

According to the present invention, the mouthpiece cover, whose recessed parts on the inside surface of said mouthpiece cover engage with the two ends of the drive gear through a male-female connection such that the inside lock connection is provided between the mouthpiece cover and the drive gear. In each actuation of the inhalation device, the gear mechanism is triggered by the rotational movement of said mouthpiece cover from the first position to the second position along the rotational path restricted on both ends by the protruding parts on the upper and the lower housing members to provide the blister package to be advanced. In each actuation of the device, the constant-distance path that the protrusions of the upper and the lower housing members define allows said mouthpiece cover to be rotated by a fixed angle. Accordingly, because of the inside lock connection provided between the mouthpiece cover and the drive gear, in each actuation of the inhalation device, only rotation of said mouthpiece cover from the first position to the second position on the constant-distance path guarantees that the blister package is advanced by the same distance and the opened blister is at accurate position so that the sufficient amount of the dry powder formulation contained in the opened blister can be inhaled. In addition to this, the patient can make sure about whether the blister is opened completely by controlling the position of the mouthpiece cover on the constant-distance path. In other words, if the mouthpiece cover is in the second position wherein it resides on the protruding part on the other end of the constant-distance rotational path, the patient makes sure that one blister is completely opened and one dose of the dry powder medicament contained in the opened blister becomes ready for inhalation; if the mouthpiece cover is between the first position and the second position, the patient makes sure about that one blister is not completely opened.

Two ends of the drive gear has been designed such that they form an inside lock connection with the mouthpiece cover. The inside lock connection between the two ends of the drive gear is formed preferably by male-female connections on the both sides. According to the present invention, due to this inside lock connection, the rotational movement of the mouthpiece cover is transmitted to the gear mechanism with minimum margin of error for the blister package to be accurately positioned. Therefore, the rotational movement of the mouthpiece cover has to be transmitted to gear mechanism exactly in order to prevent uncontrolled dosing resulting from the mispositioning of the blister package when the inhalation device is actuated. The mouthpiece cover and the drive gear synchronize with maximum ±0,01% margin of error thanks to the inside lock connection between the mouthpiece cover and the drive gear. This rate of margin of error resulting from the connection between the mouthpiece cover and the drive gear enables each blister pocket from the first one to the last to be opened completely because the precise positioning of the blister package is based on exact transmission of the movement of the mouthpiece cover to the other components in an inhalation device triggered by the mouthpiece cover. Accordingly, the drive gear, which has an inside lock connection with the mouthpiece cover, transmits the movement of the mouthpiece cover that rotates by the same angle in each inhalation to the indexing wheel via an indexing ratchet wheel interlocked into an indexing wheel and enables the blister package to be indexed properly and the opened blister pocket to be positioned accurately in the device pertaining to the present invention.

According to the present invention, the indexing wheel can be another component of the gear mechanism. As a result of the transmission of the rotation of the mouthpiece cover to the gear mechanism via the drive gear with ±0.01% margin of error, the blister pockets are received in the recesses of the indexing wheel, the blister package is indexed properly and the opened blisters are accurately positioned. The recesses of the indexing wheel match with the shape of the blister package. Blister pockets of the blister package are received in these recesses while the indexing wheel rotates. The rotation angle of the indexing wheel depends on the number of the recesses of the indexing wheel. In each actuation of the inhalation device, the indexing wheel can be rotated by an angle of 15° to 120°. According to the invention, there are preferably 8 recesses of the indexing wheel. Therefore, the indexing wheel is supposed to rotate 45 degrees when the device is actuated for the blister to be positioned accurately. In the device pertaining to the present invention, the rotational movement of the mouthpiece cover which is fixed at a value between 30° and 160° is transmitted to the indexing wheel with ±0.01% margin of error owing to the connection between the mouthpiece cover and the drive gear whenever the device is actuated. Therefore, the indexing wheel rotates 45 degrees whenever the device is actuated. As the indexing wheel rotates 45 degrees whenever the device is actuated, the blister package is accurately positioned and controlled dosing of the medicament in dry powder from is provided.

At least one component may be situated on the lower housing member and serves as a stopper interlocks with the tooth of at least one of the gears of the gear mechanism and stabilizes the gear in a suitable position in order to prevent backward rotation of the blister package and enables it to be precisely positioned. While the stopper can hinder the rotation of any gear of the gear mechanism, it preferably hinders the rotation of the indexing ratchet wheel interlocked in the indexing wheel. The stopper can be anywhere suitable in the lower housing member and have any shape.

The counter gear in the device pertaining to the present invention may display the number of the unused blister pockets remained in the device. In response to the actuation of the device by the mouthpiece cover, the mouthpiece is uncovered, the blister package is indexed and one dose of the dry powder medicament is prepared for inhalation while the counter gear rotates as well. Thus, the movement of the mouthpiece cover leads to the mouthpiece being exposed; one dose of the dry powder medicament to be ready for inhalation after the blister pocket is opened as well as providing the counter gear to rotate and display the new value of the remaining unused blister pockets through the display aperture.

On the counter wheel, there exist numerals equal to the number of the blister pockets present in the device and they are spaced by equal angles. In a device comprising 60 doses, the angle between the numerals is approximately 5 degrees. The counter gear rotates as a result of the reflection of the rotation of the indexing wheel gear via the pinion gear and the base gear. In response to the each actuation of the device, rotation of the indexing wheel by the same angle each time due to the accurate transmission of the movement of the mouthpiece cover to the gear mechanism via the drive gear results in the rotation of the counter gear approximately by the same angle as well, and each numeral

on the counter wheel is clearly seen through the display aperture on the upper housing member. Therefore, the patient is sure about the number of the unused blister pockets remaining in the device.

Before the inhalation, the mouthpiece cover is rotated from one end to the other and the drive gear having an inside lock connection with the mouthpiece cover transmits the movement of the mouthpiece cover to the indexing ratchet wheel with maximum ±0.01% margin of error in order to prepare the dry powder medicament for inhalation. The indexing ratchet wheel interlocks with the indexing wheel from inside because of its arms and enables the indexing wheel to rotate 45 degrees in each actuation of the device. Upon the indexing wheel's movement, the blister package is indexed and it is peeled by means of the beak present in the housing. As the indexing wheel gear engages with a winding wheel gear and the pinion gear, these gears move with the indexing wheel's movement, too. Since the mechanism wheel engages both with the winding wheel gear and a winding wheel, the rotation of the winding wheel gear causes the winding wheel to move and the lid sheet of the blister package coils on the winding wheel tightly. The pinion gear engages with the base gear while the small gear under the base gear engages with the counter gear. In brief, with the indexing wheel's rotation, both the blister package indexes and coils on the winding wheel, and the counter gear rotates 5 degrees and the number of the unused blisters remaining in the device is clearly seen through the display aperture.

The inhaler according to the present invention comprises a blister package composed of a plurality of blister pockets each of which comprises medicament in dry powder form and which are spaced at equal intervals. The blister package carries the medicament in dry powder form in one-dose portions and it is preferably a blister strip and it is preferably peelable. The blister pockets comprised in the blister package are spaced in equal intervals and each of them carries one dose of the medicament in dry powder form. While the blister package is indexed on the indexing wheel, the beak on the housing peels the blister. Therefore, one dose dry powder medicament becomes ready for inhalation after the blister package is peeled open in each actuation of the device.

The base sheet of the blister package on which the blister cavities are spaced is accumulated in the separated compartment of the housing. The lid sheet that provides impermeability of the blister package, on the other hand, is coiled on the winding wheel which is one of the components of the gear mechanism positioned on another side of the housing. The winding wheel has a plurality of resilient wings. In each actuation of the device, the movement of the winding wheel is enabled by the rotation of the winding wheel gear, which the winding wheel communicates with in each actuation of the device, by the indexing wheel. The mechanism wheel that engages both with the winding wheel and the winding wheel gear causes the winding wheel to move unidirectionally.

According to another aspect, the blister opened by the beak may be situated immediately under a manifold. The airflow that enters the device through at least one air inlet on the upper housing member entrains the dry powder medicament in the opened blister pocket via the manifold to the mouthpiece and enables the delivery of said medicament to the patient. The air inlet on the upper housing member that allows the entry of air can be in any suitable shape and size that enables external air to enter the device easily and at a convenient speed.

The air inlet that the external air flow passes through is designed not to be close where the patient holds the device in order not to prevent air flow. Furthermore, in order to deliver the required amount of the dry powder medicament in the opened blister to the patient, the air inlet is designed such that it allows the entry of the airflow through the air inlet at a convenient angle.

One end of the manifold communicates with the opened blister while the other end communicates with the inlet. On the end of the manifold that communicates with the blister, at least two apertures with four sub-apertures are situated. Upon the inhalation by patient, some of the air flow which enters through the air inlet passes through one of these apertures and; entrains the medicament in dry powder form through the other aperture to the manifold. There is preferably a tapered channel between the manifold and the mouthpiece to connect these components each other. The medicament in dry powder form that is entrained to the manifold with the airflow is delivered to the patient via the tapered channel and the mouthpiece. The manifold can be in appropriate shape through it is preferably longer than 1mm.

The mouthpiece is designed to fit the mouth for the patient to comfortably inhale the medicament in dry powder form. According to the shape of the device, the mouthpiece can be in any suitable shape or size as well as being fixed or movable. Furthermore, it could be attached or unattached to the upper and/or the lower housing members.

Each component of the device pertaining to the present invention can be made of any suitable material through they are preferably made of plastics material. These plastics can preferably be chosen from a group comprising the types of styrene acrylonitrile, polyoxymethylene acetale, acrylic-polymethylmetacrylate, cellulose acetate, polyetheretherketone, polyvinyl choloride, polyethylene, polypropylene, acrylonitrile butadiene styrene, polycarbonate, polyamide, polystyrene, polyurathane or fluoropolymer while it is preferably polyoxymethylene acetale. The plastic components can be manufactured by methods like injection molding. Moreover, each component can be in any suitable color.

The lid and the base sheets constituting the blister package preferably consist of a plurality of layers. These layers are preferably chosen from a group comprising polymeric layers that are made of polymeric substances, aluminum foil and fluoropoylmer film.

According to the present invention, the lid and base sheets forming the blister package are sealed very tightly by at least one of the methods preferably comprising cold formed bonding, hot metal bonding, hot metal welding, radio frequency welding, laser welding or ultrasonic welding in order to provide impermeability, more preferably by cold formed bonding method. Since these cold formed bonding methods can be carried out at lower temperatures than hot sealing methods, they are the most appropriate methods to use in the case that the medicament carried in the blister is heat sensitive.

Fluoropolymer film is a polymeric film which is used in blister packs and provides an excellent moisture barrier. This chemically inert polymeric film does not cause any change in the taste of the formulation when it is in contact with the dry powder formulation. In addition, it easily constitutes a layered structure with the other polymeric layers which are composed of various polymers. It is appropriate to be transacted with heat.

For preserving the stability of the dry powder formulation stored in the blister package, preferably at least one of the polymeric layers comprises at least one desiccant agent including silica gel, zeolite, alumina, bauxite, anhydrous calcium sulfate, activated carbon and clay which has the property of water absorption in order to decrease gas and moisture permeability of the layer.

In order to provide high moisture and gas protection, aluminum foil can be used in the lid and base sheets of blister packs. This aluminum foil must be thick enough to provide the desired protection for the stability of the moisture sensitive dry powder formulation stored in the blister cavity and it may be preferably in the range of 5 to 80 µm, more preferably in the range of 15 to 65 µm.

The polymeric layers in the lid and the base sheets of the blister pack mentioned in the present invention are made of the same or different polymers. The thickness of these polymeric layers varies according to the type of the polymeric substance used and its properties and are preferably in the range of 5-100 µm, more preferably in the range of 15-60 µm.

In the blister package, the layer which is in contact with the dry powder medicament in the blister cavity is preferably a polymeric layer. Due to the porous structure of aluminum foil and electrostatic forces, some of the dry powder formulation adheres onto the inside surface of the blister cavity and hence, this may cause uncontrolled dosing.

The term "uncontrolled dosing" refers to the intake of the medicament in dry powder form less or more than the required amount.

The polymers composing the polymeric layer are preferably selected from thermoplastics such as polyethylene, polypropylene, polystyrene, polyolefin, polyamide, polyvinyl chloride, polyurethane or synthetic polymers.

The blister pockets in the blister package can be in any appropriate shape. The plurality of blister pockets spaced at equal intervals on the base sheet of the blister package can be in the same or different shape, structure or volume.

The reference numbers of the drawings added to exemplify the present invention and the detailed description of the invention according to these drawings are given below but the scope of the invention should not be limited to these drawings.

### In the Drawings

Figure 1 is a perspective view of an inhaler according to the present invention;
Figure 1a and figure 1b is a view of the A-A and B-B cross sections of the inhaler of the present invention;
Figure 2 is an exploded view of the inhaler of the invention;
Figure 3 is a perspective view of the blister pack for use with the inhaler of the invention;
Figures 4a and 4b are perspective plan and bottom views of the housing of the inhaler according to the invention respectively;
Figures 5a and 5b are perspective views of the inner and outer sides of the upper housing member of the inhaler according to the invention respectively;
Figures 6a and 6b are perspective views of the inner and outer sides of the lower housing member of the inhaler according to the invention respectively;
Figures 7a and 7b are perspective views of the inner and outer sides of the mouthpiece cover of the inhaler according to the invention respectively;
Figures 8a and 8b are perspective views of the drive gear of the inhaler according to the invention;
Figure 9 is an exploded view of the connection between the drive gear and the mouthpiece cover;
Figure 10 is a cross-sectional view of the blister package delaminating in course of the operation of the inhaler of the present invention;
Figure 11 is a cross-sectional view of the engagement of the gears composing the gear mechanism in the inhaler of the present invention;
Figure 12 is a cross-sectional view of the engagement of the gears composing the gear mechanism in the inhaler of the present invention;
Figure 13 is a perspective view of the counter gear used in the inhaler of the present invention.

### Detailed Description

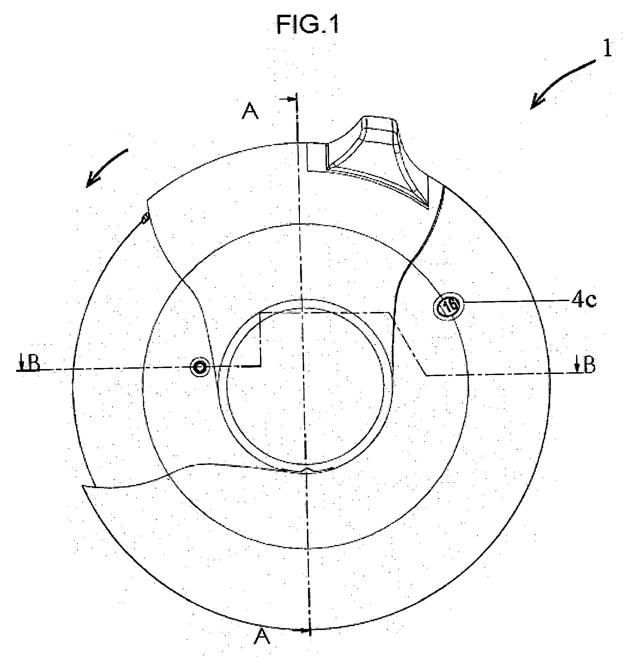

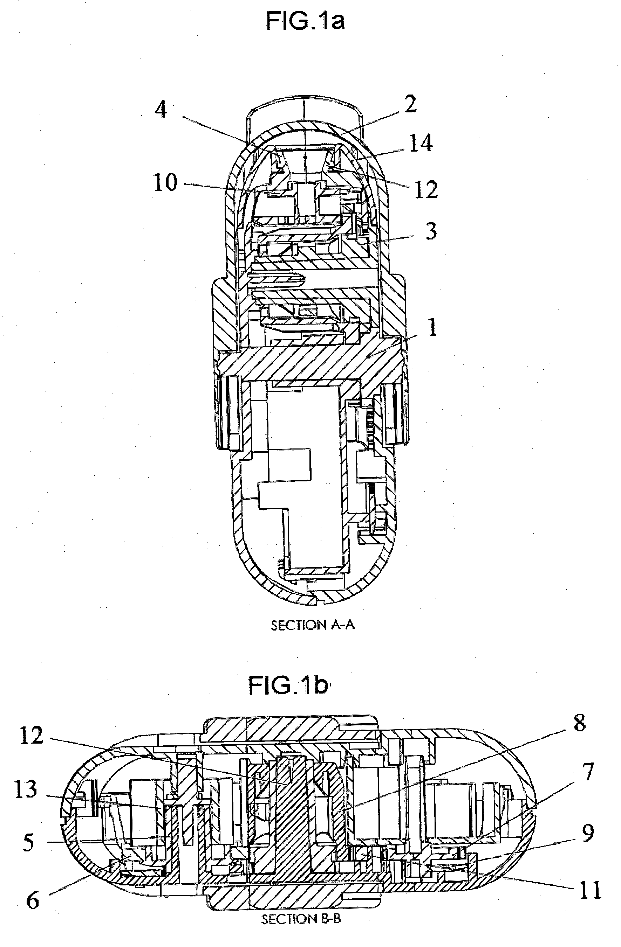

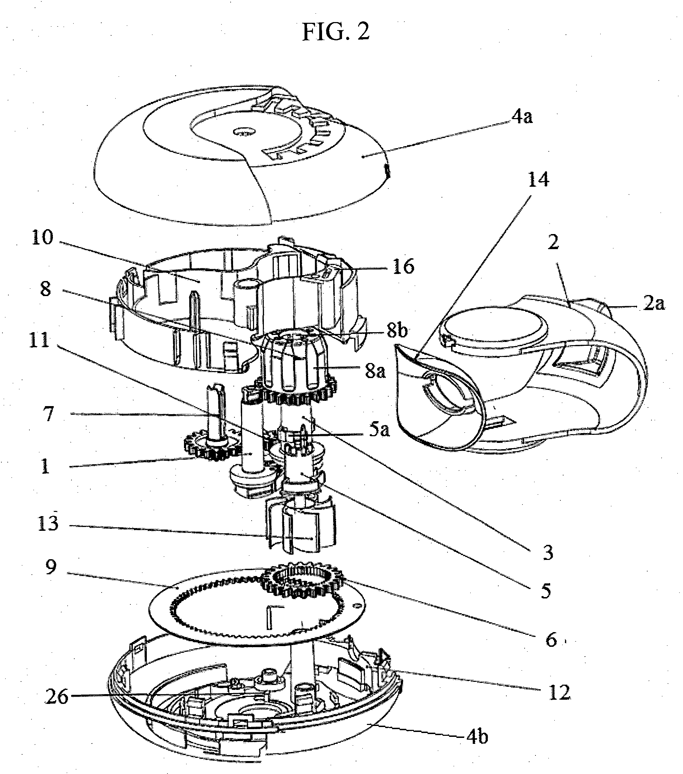

An inhaler (1) according to the present invention comprises a gear mechanism situated on a housing (10) between an upper housing member (4a) and a lower housing member (4b) in order to enable the inhalation of the dry powder medicament carried in a blister package as displayed in Figure 1a, 1b and 2. Each component of the gear mechanism is positioned at particular spots on the housing (10) to guarantee proper and accurate working. The cross-sectional view A-A in Figure 1a and the cross-sectional view B-B of Figure 1b clearly display the communication of the gear mechanism with the other components of the inhaler (1) and their locations.

The inhaler (1) of the present invention shown in Figure 1 is ready for use. In this case, a mouthpiece cover (2) is in a first position and completely covers a mouthpiece (14). The mouthpiece cover (2) has to be rotated as indicated in figure 1 by means of the finger tab (2a) on the mouthpiece cover (2) in order to activate the second position wherein the mouthpiece (14) is completely exposed. In this way, the mouthpiece (14) is exposed upon the mouthpiece cover's (2) rotation as indicated in figure 1 and the gear mechanism is triggered via the drive gear (12) which has an inside lock connection with the mouthpiece cover (2). The drive gear (12) transmits the movement of the mouthpiece cover (2) to the indexing ratchet wheel (3).

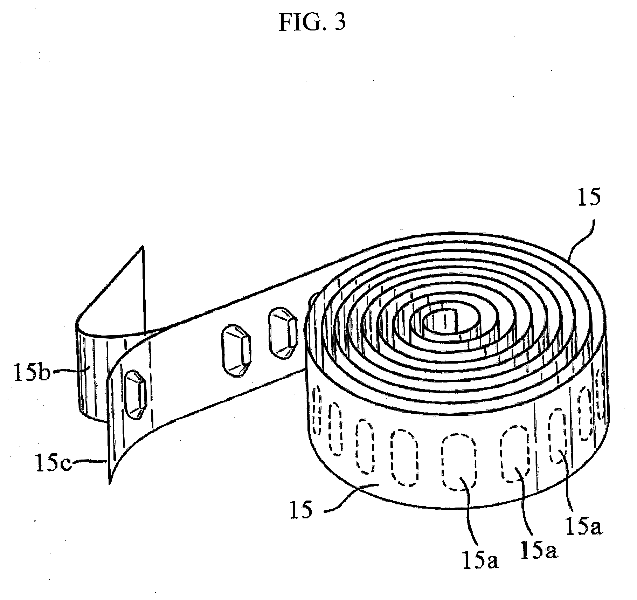

An indexing wheel (8) engages with an indexing ratchet wheel (3) and enables a blister package (15) shown in figure 3 to be indexed. Blister pockets (15a) composing the blister package are received in recesses (8a) on the indexing wheel and the blister package (15) is indexed when the indexing wheel (8) rotates. According to this, shapes of the recesses (8a) on the indexing wheel (8) have been designed to be matching with the shapes of the blister pockets (15a) composing the blister package (15) for the blister package to be indexed properly.

The blister package (15) shown in figure 3 is composed of a lid sheet (15b) which provides impermeability and a base sheet (15c) on which the blister pockets (15a) are spaced at equal intervals. Each blister pocket contains medicament in dry powder form comprising one or more active agents.

The rotational movement that the mouthpiece cover (2) of the device executes so as to actuate the inhaler while switching from the first position to the second is transmitted to the indexing ratchet wheel (3) via the drive gear (12) that the mouthpiece cover (2) engages with. As displayed in figure 2, arms of the indexing ratchet wheel (3a) interlock with protrusions (8b) inside the indexing wheel (8) and rotate the indexing wheel (8) unidirectionally. Therefore, the blister package (15) is indexed forward while the indexing wheel (8a) rotates as the blister pockets (15a) composing the blister package (15) are received in the recesses (8a) of the indexing wheel. The beak (16) in the housing (10) peels the blister package (15) while the blister package (15) is indexed and provides one blister pocket (15a) to be opened in response to each actuation of the device (1).

A winding wheel gear (6), which is another component of the gear mechanism, engages with the indexing wheel (8). As can be seen in figure 2, a mechanism wheel (5), which interlocks with the winding wheel (13) from inside, has arms (5a) to interlock with the interior teeth of the winding wheel gear (6). Therefore, the mechanism wheel (5) engages with both the winding wheel (13) and the winding wheel gear (6) and moves with these component in actuation of the inhaler (1). When the indexing wheel (8) rotates the winding wheel gear (6) upon the actuation of the inhaler (1), the winding wheel (13) rotates unidirectionally owing to the arms (5a) of the mechanism wheel which interlocks with the interior teeth of the winding wheel gear (6) and the lid sheet (15b), which is peeled away by the beak (16) while the blister package is indexed, is tightly coiled on the wings (13a) of the winding wheel. The base sheet (15c) of the blister package (15) where the blister pockets are spaced is accumulated in a separate part of the device.

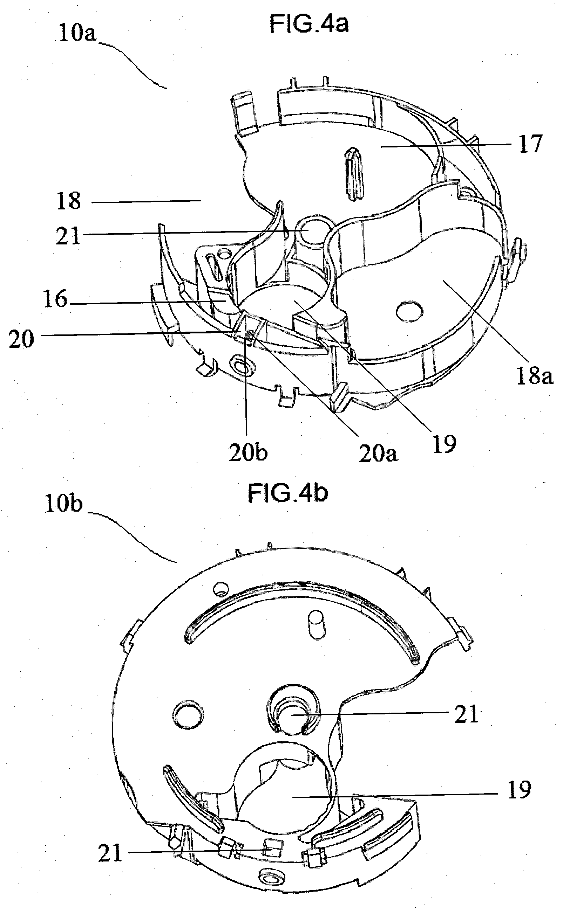

A plan view (10a) and bottom view (10b) of the housing (10) wherein the gear mechanism and the other components of the inhaler (1) pertaining to the present invention are arranged are displayed in figures 4a and 4b. Furthermore, as can be seen in figures 4a and 4b, the housing (10) also comprises components having significant roles in the actuation of the device such as the beak (16), the manifold (20), the apertures with four sub-apertures (20a, 20b). Each component comprised in the housing is situated in a separate part of the housing (10) in order to enable the inhaler (1) to work properly. The drive gear (12) passes through the center-piece (21) of the housing and joins the mouthpiece cover (2) at two points. The blister package (15) is in the coiled up lower part (17) of the housing. In response to each actuation of the device (1), the blister package (15) is peeled by the beak (16) in the housing while being indexed by the indexing wheel (8) situated in the upper part (19) of the housing. The lid sheet (15b) of the blister package (15), which provides impermeability, is indexed over the beak (16) and coiled on the wings of the winding wheel (13a) which is situated in the side part (18) of the housing. The base sheet (15c) of the blister package (15) on which the blister pockets (15a) are spaced, on the other hand, is accumulated in the separated compartment (18a) of the housing (8). Upon the inhalation by the patient, the air passes through the aperture with four sub-apertures (20a) in the lower part of the manifold (20) into the opened blister pocket. The air entrains the dry powder medicament contained in the opened blister pocket (15a) in response to each actuation of the device; and passes through the other aperture with four-sub-apertures (20b) and reaches the mouthpiece via the manifold (20).

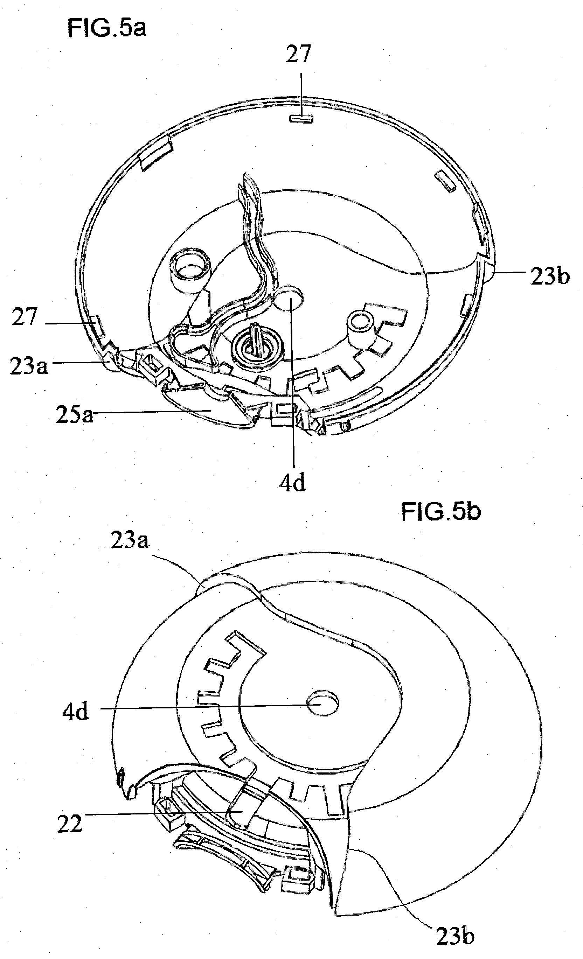

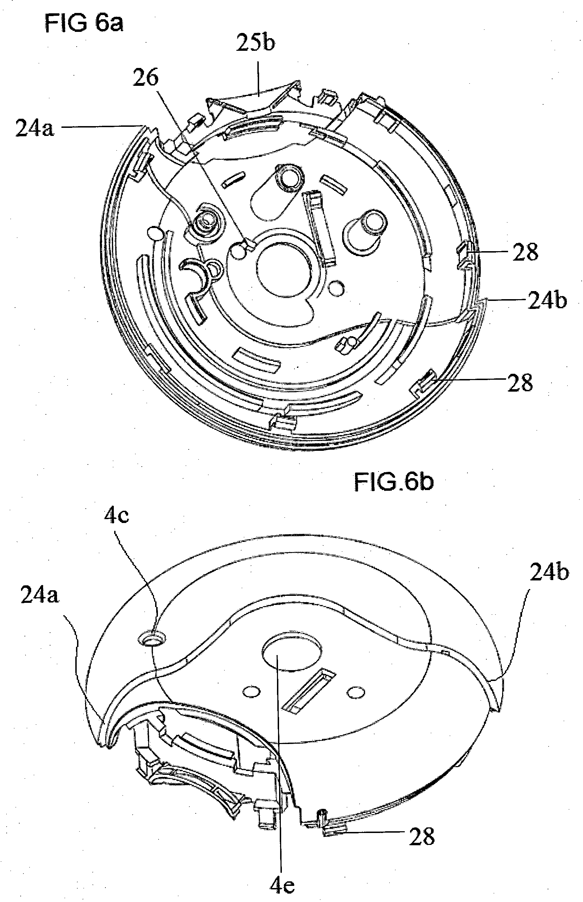

The housing (10) and the other components of the inhaler (1) pertaining to the present invention are kept together as upper housing member (4a) displayed in figures 5a and 5b and lower housing member (4b) displayed in figures 6a and 6b are joined together. Engagement tabs (28) on the inside surface of the lower housing member (4b) engage with engagement recesses (27) on the inside surface of the upper housing member (4a) and the upper and lower housing members are fixed tightly. Therefore, the protrusions (23a, 23b) on the upper housing member (4a) and the protrusions (24a, 24b) on the lower housing member (4b) are joined end to end and define a restricted path for the rotational movement of the mouthpiece cover (2). In each actuation of the device, the constant-distance path that the protrusions (23a, 23b, 24a, 24b) of the upper and the lower housing members (4a, 4b) define allows said mouthpiece cover (2) to be rotated by a fixed angle.The mouthpiece cover (2) can be easily moved from the first position to the second position along this restricted path. When the mouthpiece cover (2) is in a first position, the mouthpiece (14) is completely covered, the device is ready for use and the mouthpiece cover (2) leans on a first protrusion (23a) on the upper housing member and the first protrusion (24a) on the lower housing member. The mouthpiece (14) is slid along the rotational path with the help of the finger tab (2a) to switch to the second position. The mouthpiece is completely exposed when the cover is in this position, one dose of the dry powder medicament is ready for inhalation and the mouthpiece cover (2) leans on the second protrusion (23b) on the upper housing member and the second protrusion (24b) on the lower housing member.

The drive gear (12) passes through the aperture (4d) in the center of the upper housing member displayed in figures 5a and 5b and the aperture (4e) in the center of the lower housing member, then it is joined with the mouthpiece cover (2) on two ends. There is a tapered channel between the manifold (20) and the mouthpiece (14). A half of this tapered channel (25a) is situated on the upper housing member (4a) and the other half of this tapered channel (25b) is situated on the lower housing member (4b). Therefore, the tapered channel between the mouthpiece (14) and the manifold (20) comprised in the housing (10) is arranged by engaging the upper housing member (4a) and the lower housing member (4b). In addition, the external air enters through at least one air inlet (22) on the upper housing member upon the inhalation of the patient, reaches the opened blister and entrains the dry powder medicament from the blister to the mouthpiece.

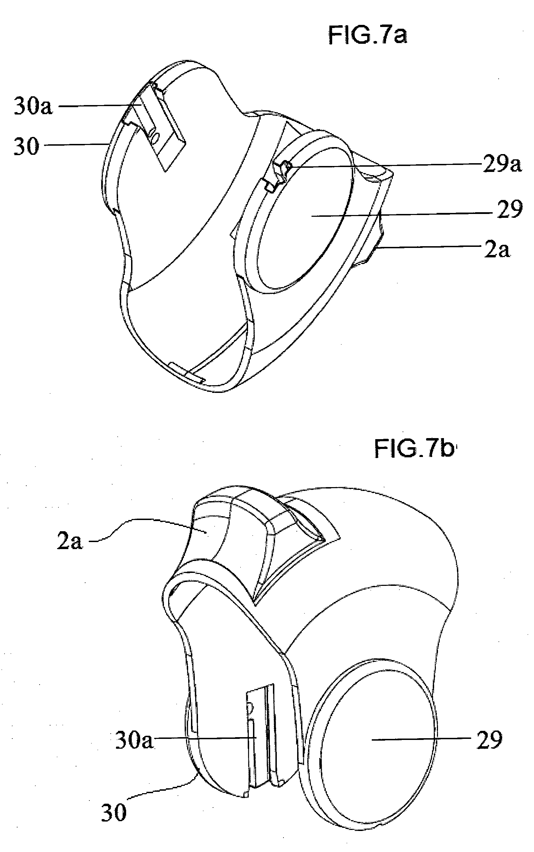

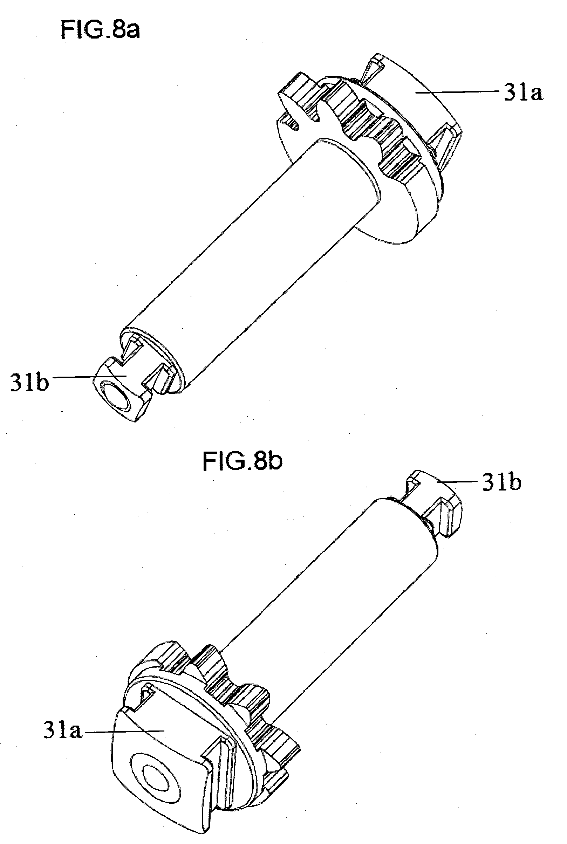

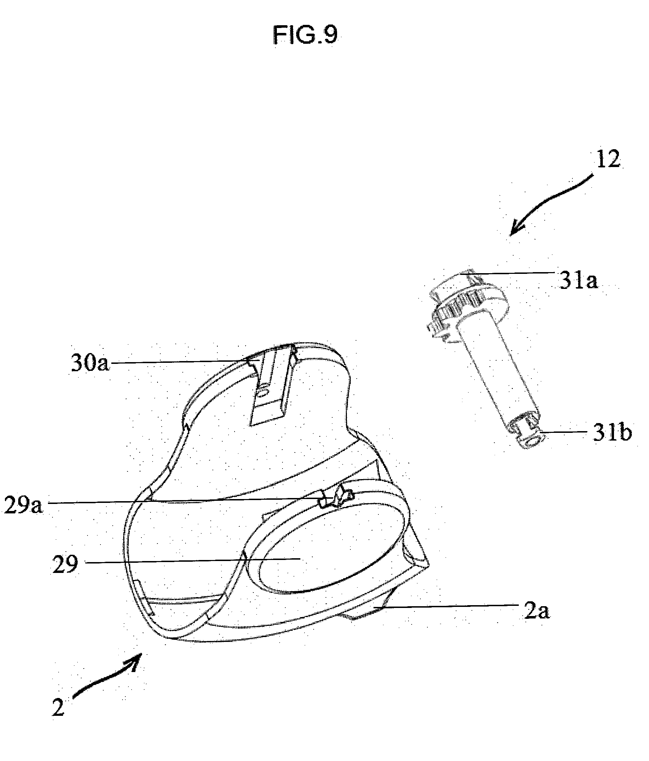

The mouthpiece cover (2) as displayed in figures 7a and 7b can be moved easily with the help of the finger tab (2a) on it. This finger tab (2a) has been designed to match finger shape and is situated in any suitable spot on the mouthpiece cover (2). As can be seen clearly in figures 7a and 7b, the first and the second connection points (29, 30) of the mouthpiece cover have recesses (29a, 30a) on their inside surfaces. Protrusions (31a, 31b) on the two ends of the drive gear (12) displayed in figures 8a and 8b make a male-female connection with these recesses (29a; 30a). The first recess (30a) on the inside surface of the mouthpiece cover engages with the first protrusion (31a) on the one end of drive gear while the second recess on the inside surface of the mouthpiece cover (29a) engages with the second protrusion (31 b) on the other end of the drive gear and the connection provided between each end of the drive gear (31a; 31b) and the corresponding recess (30a; 29a) on the inside surface of the mouthpiece cover is a male-female connection. The male-female connection that each end of the drive gear (31 a; 31b) makes with the corresponding recessed parts on the inside surface of the mouthpiece cover (30a; 29a) causes to form an inside lock connection between the drive gear (12) and the mouthpiece gear (2) with maximum ±0,01% margin of error. This is clearly displayed in figure 9. Therefore, the rotational movement of the mouthpiece cover (2) on the upper (4a) and lower (4b) housing members is accurately transmitted to the indexing ratchet wheel by the drive gear (12) which has an inside lock connection with the mouthpiece (2) on its two ends.

The mouthpiece cover (2) rotates by the same angle each time it is switched from the first position to the second position on the path restricted by the protrusions (29a, 29b, 30a, 30b) on the upper and the lower housing members (4a, 4b). The rotational angle of the mouthpiece cover (2) varies depending on the shape and the size of the device but is a fixed value between 30° and 160°. This angle is adjusted according to the shape and the size of the device such that the indexing wheel (12) having 8 recesses (8a) rotates 45 degrees in response to each actuation of the device. The mouthpiece cover (2) rotates by the same angle on its two ends in response to each actuation of the device and this rotational movement of the mouthpiece cover (2) is accurately transmitted to the indexing wheel (12) by the indexing ratchet wheel (3) because of the drive gear (12) which is tightly attached to the mouthpiece cover (2) and the indexing wheel is provided to rotate 45° each time the device is triggered.

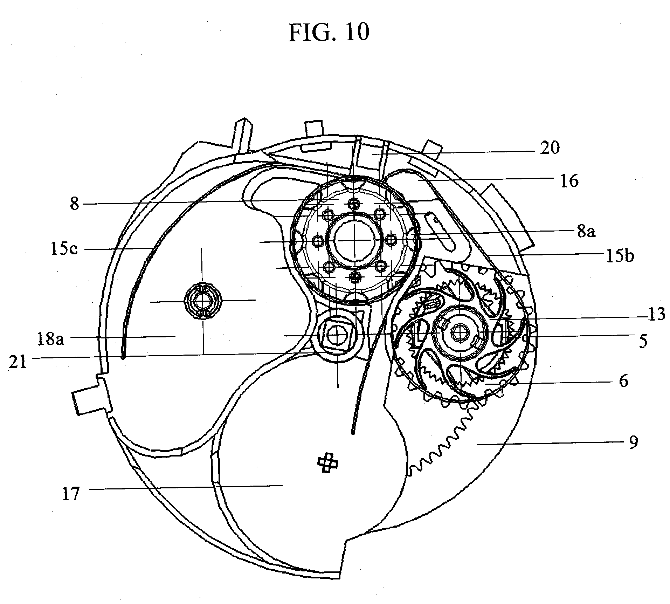

There are 8 recesses (8a) on the indexing wheel displayed in figure 2 and the indexing wheel (8) rotates 45° each time for the blisters received in these recesses to be able to be positioned accurately. The blister package (15) is indexed by the 45° rotation of the indexing wheel (8) in response to each actuation of the device and is peeled by the beak (16) so one dose of the dry powder medicament becomes ready for inhalation when one blister pocket is opened. As seen in figure 10, the lid sheet (15b) that is peeled away by the beak (16) and the base sheet (15c) of the blister package (15) are enclosed in separate compartments. The lid sheet (15b), which provides impermeability of the blister package, passes over the beak (16) and tightly coils on the wings (13a) of the winding wheel.

The base sheet (15c) with blister pockets (15a), each of which comprises one dose of the dry powder medicament, is accumulated in the separated compartment (18a) in the housing (10). In response to each actuation of the device (1), one dose of the dry powder medicament becomes ready for inhalation after one blister pocket (15a) is opened; the air which enters the device through the air inlet (22) upon the inhalation of the patient entrains the dry powder medicament to the mouthpiece and provides to deliver it to the patient.

There is at least one stopper (26) in the lower housing member displayed in figure 6a in order to provide the opened blister in the blister package (15) which is indexed by the indexing wheel (8) so as to be positioned accurately. This stopper (26) engages with the tooth of at least one gear and provides the position of this gear to stay immovable and the opened blister to be accurately positioned. This stopper (26) can be any suitable shape and size according to the structure of the gear it stops.

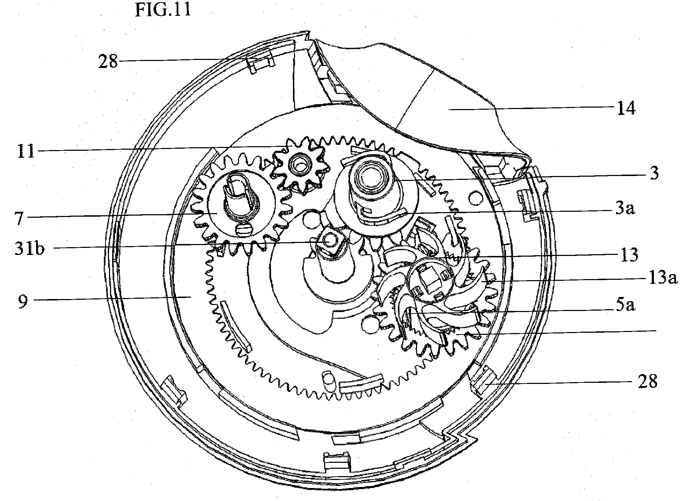

Figure 11 shows that the stopper (26) engages with the teeth of the indexing ratchet wheel (3) and stops it. The rotational movement of the mouthpiece cover (2) by the same angle in response to each actuation of the device (1) is accurately transmitted to the indexing ratchet wheel (3) by the drive gear (12), which has an inside lock connection with the mouthpiece cover (2) in its both ends, and the indexing ratchet wheel is provided to be rotated by approximately the same angle each time the indexing wheel (8) is triggered. The stopper component (26) in the lower housing member (4b) prevents the backwards movement of the blister package (15) indexed by the indexing wheel (8), which synchorizes with the indexing ratchet wheel by keeping the position of the indexing ratchet wheel (3) fixed and provides the blister package (15) to be precisely positioned.

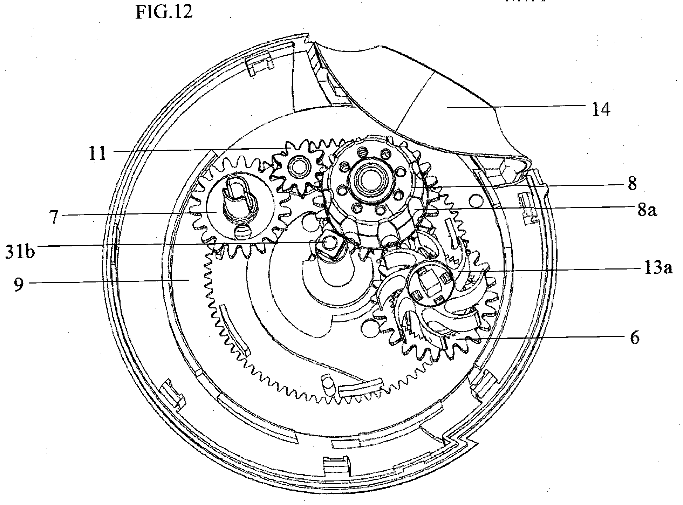

As can be seen in figure 12, the indexing wheel (8) which synchronizes with the indexing ratchet wheel (3) is engaged with the winding wheel gear (6) and the pinion gear (11) and the rotation of the indexing wheel (8) causes the pinion gear (11) and the winding wheel gear (6) to rotate. Thus, both the peeled lid sheet (15b) of the blister package (15), which is indexed by the rotation of the indexing wheel (8), is tightly coiled on the winding wheel (13), engaging with the winding wheel gear (6) via mechanism wheel (5), and also the counter gear (9) is moved by the pinion gear (11) and the base gear (7) as a result of the rotation of the indexing wheel (8).

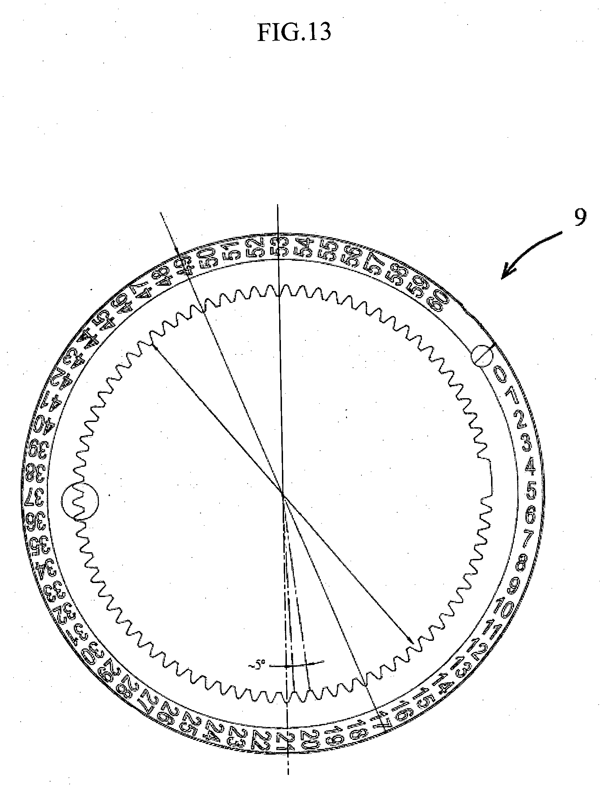

The movement of the indexing wheel (8) is transmitted to the base gear (7) engaging with the pinion gear (11) by the pinion gear (11). The small gear which is under the base gear (7) and attached to it engages with the counter gear (9). Thus, the movement of the indexing wheel (8) is transmitted to the counter gear (9) shown in figure 13 by the pinion gear (11) and the base gear. There are numerals incrementing from 1 to 60 in the counter gear (9) displayed in figure 13. The angles between these numerals are all equal and approximately 5 degrees. The movement of the indexing wheel (8) by 45 degrees in response to each actuation of the device causes the counter gear to move approximately 5 degrees and the number of the unused blister pockets remained in the device to be clearly seen through the display aperture (4c) on the lower housing member (4b).

Use of the device described in figures 1-13, express the mouthpiece (14) when the mouthpiece cover (2) is slid from the first position to the second position on the rotational path restricted on both ends by the protruding parts (23a, 23b, 24a, 24b) on the upper housing member (4a) and the lower housing member (4b); the gear mechanism is triggered by the rotational movement of the mouthpiece cover (2) via the drive gear (12) and one dose of dry powder medicament is prepared for inhalation; the counter gear (9) is indexed and the numeral seen through the display aperture (4c) on the lower housing member (4b) is incremented. After inhalation, the mouthpiece cover (2) is moved from the second position to the first position wherein the mouthpiece (14) is completely covered.

The medicament in dry powder form which is stored in blister cavities is manufactured according to the prior art. According to the present invention, the particle sizes of the active agents comprised in the dry powder medicament are smaller than 20 µm, preferably smaller than 10 µm.

The inhaler utilized in the present invention has been designed so as to deliver the dry powder medicament used in monotherapy or combined therapy. The term "monotherapy" refers to inhalation treatments in which dry powder medicaments comprising a single active agent are used whereas the term "combined therapy" refers to inhalation treatments in which dry powder medicaments comprising more than one active agents are used.

The dry powder medicament delivered via the device of the present invention comprises at least one excipient in addition to the active agent or agents. These excipients are generally chosen from a group comprising monosaccharides (glucose, arabinose, etc.), disaccharides (lactose, saccharose, maltose, etc.), oligo- and polysaccharides (dextran, etc.), polyalcohols (sorbite, mannite, xylite), salts (sodium chloride, calcium carbonate, etc.) or combinations thereof. The medicament in dry powder form comprises fine or coarse excipients particles preferably having various particle size ranges in order to deliver the required amount to the lungs.

The active agents comprised in the dry powder medicament which is stored in blister packages used in the device pertaining to the present invention can be selected from a group comprising cromolyns, anti-infectives, antihistamines, anti-inflammatories, bronchodilators, leukotirene inhibitors, PED IV inhibitors, antitussives, diuretics, anticholinergics, hormones, xanthines and pharmaceutically acceptable combinations thereof.

The active agent comprised in the medicament in dry powder form delivered via the inhaler pertaining to the present invention is selected from a group comprising tiotropium, oxitropium, flutropium, ipratropium, glicopironium, flunisolid, beclomethasone, budesonide, fluticasone, mometasone, ciclesonide, rofleponide, dexamethasone, montelukast, methylcyclopropane acetic acid, sodium cromoglicat, nedocromil sodium, Npropylene, teophylline, roflumilast, ariflo (cilomilast), salmeterol, salbutamol, formoterol, terbutaline, carmoterol, indacaterol, cetirizine, levocetirizine, efletirizine, fexofenadine and their racemates, free base, enantiomers or diastereomers and preferably their pharmaceutically acceptable salts, solvates and/or hydrates or a combination of said active agents.

The device pertaining to the present invention is used in administration of the medicament in dry powder from which is utilized in the treatment of many respiratory diseases such as asthma, chronic obstructive pulmonary disorder (COPD) and allergic rhinitis. Accordingly, the respiratory diseases include, but not restricted to, allergic or non-allergic asthma at any phases, acute lung injury (ALI), acute respiratory distress syndrome (ARDS), exacerbation of airways hyperactivity, bronchiectasis, chronic obstructive pulmonary including emphysema and chronic bronchitis, airways or lung diseases (COPD, COAD or COLD), pneumoconiosis, aluminosis, anthracosis, asbestosis, chalicosis, ptilosis, siderosis, silicosis, tabacosis and byssinosis. The device pertaining to the invention can be used in prophylactic or symptomatic treatment. In addition, the device pertaining to the present invention is used to deliver medicament in dry powder form which is preferably used in the symptomatic treatment of allergic asthma and COPD.

## Claims

1. An inhaler (1) suitable for delivery of the medicament in dry powder form from a blister package (15) composed of a plurality of blister pockets (15a) each of which comprises medicament in dry powder form and which are spaced at equal intervals; the inhaler comprising;
- a mouthpiece (14) enabling a patient to inhale the medicament in dry powder form from an opened blister pocket (15a) in use;
- a gear mechanism enabling, in use a blister package (15) to be indexed and the medicament in dry powder form to become ready for inhalation;
- a rotatable mouthpiece cover (2) covering the mouthpiece (14) and triggering the gear mechanism;
- a drive gear (12) which is joined with the mouthpiece cover (2); and
wherein an inside lock connection is provided between the mouthpiece cover (2) and the drive gear by two ends (31a; 31b) of the drive gear (12) which enable the mouthpiece cover (2) to connect with the gear mechanism, to engage with the recessed parts (30a; 29a) on the inside face of the mouthpiece cover through a male-female connection,
**characterised in that**, the inhaler further comprises a housing (10) situated between an upper housing member (4a) and a lower housing member (4b) in which the blister package (15) and the gear mechanism are enclosed in use.

2. The inhaler (1) according to claim 1, wherein said inhaler comprises at least one stopper (26) in the lower housing member (4b).

3. The inhaler (1) according to claim 2, wherein the stopper (26) engages with the tooth of at least one gear and hinders its rotation.

4. The inhaler (1) according to claim 2, wherein the stopper (26) hinders the rotation of an indexing ratchet wheel (3) in the gear mechanism.

5. The inhaler (1) according to any one of the preceding claims, wherein the stopper (26) engages with the teeth of the indexing ratchet wheel (3) and hinders its rotation in order to precisely position the blister package (15).

6. The inhaler (1) according to claim 1, wherein said inhaler comprises a path restricted by protrusions (23a, 23b; 24a, 24b) on the upper (4a) and the lower (4b) housing members.

7. The inhaler (1) according to claim 1, wherein the mouthpiece cover (2) moves rotationally by being slid on a restricted path on the upper (4a) and the lower (4b) housing members.

8. The inhaler (1) according to any one of the preceding claims, wherein said mouthpiece cover (2) rotates along a constant-distance path that recesses (23a, 23b; 24a, 24b) of the upper and the lower housing members define by a fixed angle in the range of 30° to 160° according to the shape of the device, in response to the each actuation of the device.

9. The inhaler (1) according to any one of the preceding claims, wherein the mouthpiece cover (2) can solely be in two positions such that:
wherein the first position, the mouthpiece cover (2) resides on a protrusion (23a, 24a) on one end of the rotational path as the mouthpiece cover is completely covered and the device is in standby mode;
wherein the second position, the mouthpiece cover (2) resides on the protrusion (23b, 24b) on the other end of the rotational path and one dose of the dry powder medicament becomes ready for inhalation in response to the actuation of the device.

10. The inhaler (1) according to claim 1, wherein said inhaler comprises a finger tab on the mouthpiece cover to move the mouthpiece cover easily.

11. The inhaler (1) according to claim 1, wherein each component contained in the gear mechanism directly or indirectly engages with each other.

12. The inhaler (1) according to claim 1 or 11, wherein the drive mechanism is comprised of:
- a drive gear (12) which provides to trigger the device (1) by transmitting the constant-angle movement of the mouthpiece cover (2) to an indexing ratchet wheel (3);
- an indexing wheel (8) which synchronizes with the indexing ratchet wheel (3) and enables the blister package (15) to be indexed in use;
- a winding wheel gear (6) which moves the winding wheel (13) via the mechanism wheel (5) upon the rotation of the indexing wheel (8);
- a pinion gear (11) and base gear (7) that transmit the movement of the indexing wheel (8) to a counter gear (9), the counter wheel (9) which displays the number of the unused blister pockets (15a) remaining in the device (1) in use.

13. The inhaler (1) according to claim 12, wherein the rotation of the indexing wheel (8) by an angle of 45° in response to each actuation of the device (1) is provided by an inside lock connection between the mouthpiece cover (2) which rotationally moves at a fixed value in the range of 30° to 160° according to the shape of the device (1).

## Patentansprüche

1. Ein Inhalator (1), der für die Abgabe des Medikaments in Form eines trockenen Pulvers aus einer Blisterverpackung geeignet ist (15), die aus einer Vielzahl von Blistertaschen (15a) besteht, von denen jede ein Medikament in Form eines trockenen Pulvers umfasst und die in gleichen Abständen beabstandet sind; der Inhalator umfasst die Folgenden;
- ein Mundstück (14), das es den Patienten ermöglicht, das Medikament in trockener Pulverform aus einer geöffneten Blistertasche (15a) im Einsatz zu inhalieren;
- ein Getriebe, das es bei Gebrauch einer Blisterverpackung (15) es ermöglicht, indiziert zu werden und, das den Medikamenten in trockener Pulverform es ermöglicht, zur Inhalation bereit zu werden;
- eine drehbare Mundstückabdeckung (2), die das Mundstück (14) abdeckt und das Getriebe auslöst;
- ein Antriebsrad (12), das mit der Mundstückabdeckung (2) verbunden ist ;und
wobei eine Innenverschlussverbindung zwischen der Mundstückabdeckung (2) und dem Antriebsrad mit zwei Enden (31 a; 31b) des Antriebsrades (12), die der Mundstückabdeckung (2) es ermöglichen, mit dem Getriebe zu verbinden und in Eingriff mit den ausgesparten Teilen (30a; 29a) auf der Innenfläche der Mundstückabdeckung durch eine männlich-weibliche Verbindung zu sein, **dadurch gekennzeichnet, dass** der Inhalator weiterhin ein Gehäuse (10) zwischen einem oberen Gehäuseelement (4a) und einem unteren Gehäuseelement (4b) umfasst, in dem die Blisterverpackung (15) und das Getriebe eingeschlossen sind.

2. Der Inhalator (1) nach Anspruch 1, wobei der genannte Inhalator mindestens einen Stopfen (26) in dem unteren Gehäuseteil (4b) umfasst.

3. Der Inhalator (1) nach Anspruch 2, wobei der Stopfen (26) in Eingriff mit dem Zahn mindestens eines Zahnrades ist und seine Drehung verhindert.

4. Der Inhalator (1) nach Anspruch 2, wobei der Stopfen (26) die Drehung eines Indizierungssperrrades (3) in dem Getriebe verhindert.

5. Der Inhalator (1) nach einem der vorhergehenden Ansprüche, wobei der Stopfen (26) in Eingriff mit den Zähnen des Indizierungssperrrades (3) ist und für die präzise Positionierung der Blisterverpackung (15) seine Drehung verhindert.

6. Der Inhalator (1) nach Anspruch 1, wobei der genannte Inhalator einen Pfad umfasst, der durch Vorsprünge (23a, 23b; 24a, 24b) auf den oberen (4a) und den unteren (4b) Gehäuseelementen eingeschränkt wird.

7. Der Inhalator (1) nach Anspruch 1, wobei die Mundstückabdeckung (2) sich dreht, indem sie auf einen eingeschränkten Pfad auf den oberen (4a) und den unteren (4b) Gehäuseelementen geschoben wird.

8. Der Inhalator (1) nach einem der vorhergehenden Ansprüche, wobei die genannte Mundstückabdeckung (2) sich entlang eines konstanten Abstandes dreht, so dass Aussparungen (23a, 23b; 24a, 24b) der oberen und unteren Gehäuseelemente um einen festen Winkel im Bereich von 30° bis 160° je nach der Form des Gerätes in Reaktion auf die jede Betätigung des Geräts definiert werden.

9. Der Inhalator (1) nach einem der vorhergehenden Ansprüche, wobei die Mundstückabdeckung (2) nur in zwei Positionen liegen kann:
Bei der erster Position befindet sich Mundstückabdeckung (2) auf einem Vorsprung (23 a, 24a) an einem Ende des Drehpfades, da die Mundstückabdeckung vollständig abgedeckt ist und das Gerät im Standby-Modus ist;
Und bei der zweiten Position befindet sich Mundstückabdeckung (2) auf dem Vorsprung (23b, 24b) am anderen Ende des Drehpfades und eine Dosis des Trockenpulvermedikaments wird als Reaktion auf die Betätigung des Geräts zur Inhalation bereit.

10. Der Inhalator (1) nach Anspruch 1, wobei der genannte Inhalator eine Fingerlasche an der Mundstückabdeckung umfasst, um die Mundstückabdeckung leicht zu bewegen.

11. Der Inhalator (1) nach Anspruch 1, wobei sich im Getriebe befindete jede Komponente direkt oder indirekt miteinander in Eingriff steht.

12. Der Inhalator (1) nach Anspruch 1 oder 11_ wobei der Antriebsmechanismus die Folgenden umfasst:
- ein Antriebsrad (12), das das Gerät (1) durch Übertragen der konstanten Winkelbewegung der Mundstückabdeckung (2) an ein Indizierungssperrrad (3) auslöst;
- ein Indizierungsrad (8), das mit dem Indizierungssperrrad (3) synchronisiert wird und der Blisterverpackung (15) es ermöglicht, im Gebrauch indiziert zu werden;
- ein Wickelzahnrad (6), das das Wickelrad (13) über den Radmechanismus (5) beim Drehen des Indizierungsrades (8) bewegt;
- ein Ritzel (11) und Basiszahnrad (7), die die Bewegung des Indizierungsrades (8) zu einem Gegenzahnrad (9) und dem Anzeigerad (9) übertragen, das die Anzahl der nicht verwendeten und im Gerät (1) verbleibender Blistertaschen (15a) zeigt.

13. Der Inhalator (1) nach Anspruch 12, wobei die Drehung des Indizierungsrades (8) um einen Winkel von 45° als Reaktion auf jede Betätigung des Geräts (1) durch eine Innenverschlussverbindung zwischen der Mundstückabdeckung (2) vorgesehen wird, die sich bei einem festen Wert im Bereich von 30° bis 160° je nach der Form des Gerätes (1) dreht.

## Revendications

1. Un inhalateur (1) approprié pour la livraison du médicament sous la forme de poudre sèche à partir d'un emballage coque (15) composée d'une pluralité de poches blisters (15a) dont chacune comprend le médicament sous la forme de poudre sèche et qui sont espacées à des intervalles égaux ; ledit inhalateur comprenant :
- un embout (14) permettant à un patient d'inhaler le médicament sous la forme de poudre sèche depuis une poche blister ouverte (15a) en cours d'utilisation ;
- un mécanisme d'engrenage permettant en cours d'utilisation un emballage blister (15) à être indexé et le médicament sous la forme de poudre sèche à être prêt pour l'inhalation ;
- une couverture rotative de l'embout (2) couvrant l'embout (14) et déclenchant le mécanisme d'engrenage ;
- un engrenage d'entrainement (12) qui est relié à la couverture de l'embout (2) ;
dans lequel une connexion de verrouillage intérieur est prévu entre la couverture de l'embout (2) et l'engrenage d'entraînement par deux extrémités (31 a, 31 b) de l'engrenage d'entraînement (12) qui permettent le cache de la couverture de l'embout (2) pour se connecter avec le mécanisme d'engrenage et à engager avec des parties évidées (30a, 29a) sur la face interne de la couverture de l'embout à travers une connexion mâle-femelle,
**caractérisé en ce que,** l'inhalateur comprend en outre, un boîtier (10) situé entre l'élément de boîtier supérieur (4a) et l'élément de boîtier inférieur (4b), dans lequel l'emballage coque (15) et le mécanisme d'engrenage sont enfermés en cours d'utilisation.

2. L'inhalateur (1) selon la revendication 1, dans lequel ledit inhalateur comprend au moins une butée (26) dans l'élément de boîtier inférieur (4b).

3. L'inhalateur (1) selon la revendication 2, dans lequel la butée (26) s'engage au dent d'au moins un pignon et cache sa rotation.

4. L'inhalateur (1) selon la revendication 2, dans lequel la butée (26) empêche la rotation de la roue programmatrice d'indexation (3) dans le mécanisme d'engrenage.

5. L'inhalateur (1) selon l'une quelconque des revendications précédentes, dans lequel la butée (26) s'engage aux dents de la roue programmatrice d'indexation (3) et empêche sa rotation afin de positionner précisément l'emballage coque (15).

6. L'inhalateur (1) selon la revendication 1, dans lequel ledit inhalateur comprend une route restreinte par les saillies (23a, 23b; 24a, 24b) sur les éléments de boîtier supérieur (4a) et inférieur (4b).

7. L'inhalateur (1) selon la revendication 1, dans lequel la couverture de l'embout (2) est déplacée par rotation par glissement sur une route restreinte sur l'élément de boîtier supérieur (4a) et inférieur (4b).

8. L'inhalateur (1) selon l'une quelconque des revendications précédentes, dans lequel ladite couverture de l'embout (2) tourne le long d'une route à distance constante que les cavités (23a, 23b; 24a, 20 24b) de l'élément de boîtier supérieur et inférieur définie par un angle fixe dans la plage de 30 ° à 160 °, suivant la forme du dispositif, en réponse à chaque actionnement du dispositif.

9. L'inhalateur (1) selon l'une quelconque des revendications précédentes, dans lequel la couverture de l'embout (2) peut être uniquement dans deux positions, telles que:
dans lequel la première position, la couverture de l'embout (2) se trouve sur une saillie (23a, 24a) sur une extrémité de la route de rotation lorsque la couverture de l'embout est complètement couvert et le dispositif est en mode veille;
dans lequel la seconde position, la couverture de l'embout (2) se trouve sur la saillie (23b, 24b) de l'autre extrémité de la route de rotation et une dose du médicament en poudre sèche devient prêt pour l'inhalation, en réponse à l'actionnement du dispositif.

10. L'inhalateur (1) selon la revendication 1, dans lequel ledit inhalateur comprend un onglet de doigt sur la couverture de l'embout pour déplacer la couverture de l'embout facilement.

11. L'inhalateur (1) selon la revendication 1, dans lequel chaque composant du mécanisme d'engrenage s'engage l'un avec les autres directement ou indirectement.

12. L'inhalateur (1) selon la revendication 1 ou 11, dans lequel le mécanisme d'entrainement est composé de :
- un engrenage d'entrainement (12) qui permet déclencher l'appareil (1) en transmettant le mouvement d'un angle constant de la couverture de l'embout (2) à une roue à rochet d'indexation (3) ;
- une roue d'indexation (8) qui synchronise avec la roue à rochet d'indexation (3) et permet l'emballage coque (15) à être indexé en cours d'utilisation ;
- un engrenage de roue d'enroulement (6) qui déplace la roue d'enroulement (13) par un mécanisme de roue (5) sur la rotation de la roue d'indexation (8) ;
- un engrenage à pignons (11) et un engrenage de base (7) qui transmettent le mouvement du la roue d'indexation (8) à une roue de contrepoussée (9) qui affiche le nombre des poches blisters (15a) inutilisées restant dans le dispositif (1) en cours d'utilisation.

13. L'inhalateur (1) selon la revendication 12, dans lequel la rotation de la roue d'indexation (8) d'un angle de 45 ° en réponse à chaque actionnement du dispositif (1) est assurée par une connexion de verrouillage intérieur entre la couverture de l'embout (2) qui se déplace en rotation à une valeur fixe dans la plage de 30 ° à 160 °, suivant la forme du dispositif (1).
